# EUROPEAN PATENT SPECIFICATION

(11) **EP 2 615 927 B1**
(45) Date of publication and mention of the grant of the patent: **02.09.2015**
(21) Application number: 11771281.0
(22) Date of filing: 14.09.2011
(51) Int. Cl.: A23L 1/03, A23L 1/30, A23L 2/02, C12R 1/225, C12R 1/25, A23L 1/212, A23L 2/38, A23L 2/52

(54) **METHOD OF PRODUCTION OF FERMENTED, PRO-HEALTHY FRUIT BEVERAGES**
VERFAHREN ZUR HERSTELLUNG VON FERMENTIERTEN GESUNDHEITSFÖRDERNDEN FRUCHTGETRÄNKEN
PROCÉDÉ POUR PRODUIRE DES BOISSONS FERMENTÉES AUX FRUITS BONNES POUR LA SANTÉ

(30) Priority: 16.09.2010 PL 39242510
(43) Date of publication of application: 24.07.2013
(73) Proprietor: Instytut Biotechnologii Przemyslu Rolno-Spozywczego, 02-532 Warszawa (PL)
(72) Inventor: OWCZAREK, Lubomila, PL-03-982 Warszawa (PL); JASINSKA, Urszula T., PL-02-206 Warszawa (PL); SKAPSKA, Sylwia, PL-02-638 Warszawa (PL)
(74) Representative: Witek, Rafal
(86) International application number: PCT/PL2011/050035
(87) International publication number: WO 2012/036575

(56) References cited:
- EP-A1- 1 508 282
- WO-A1-2005/071061
- US-A- 5 322 836
- SKAPSKA S. ET AL.: "Zmiany pojemnosci przeciwutleniajacej grzybow jadalnych w procesie kiszenia", ZYWNOSC. NAUKA. TECHNOLOGIA. JAKOSC, vol. 4, no. 59, 2008, pages 243-250, XP002664097,
- OGAWA ET AL: "Production of conjugated fatty acids by lactic acid bacteria", JOURNAL OF BIOSCIENCE AND BIOENGINEERING, ELSEVIER, AMSTERDAM, NL, vol. 100, no. 4, 1 October 2005 (2005-10-01), pages 355-364, XP005667209, ISSN: 1389-1723, DOI: 10.1263/JBB.100.355
- KISHIMOTO NORIAKI ET AL: "Two distinct pathways for the formation of hydroxy FA from linoleic acid by lactic acid bacteria.", LIPIDS, vol. 38, no. 12, December 2003 (2003-12), pages 1269-1274, XP002664098, ISSN: 0024-4201

## Description

Subject of the invention is the method for production of fermented, prohealthy fruit beverages as replacers of fermented milks - especially for persons following the appropriate elimination diets.

The process of lactic fermentation is most commonly applied worldwide for production of beverages based on milk and other dairy products. However, these products are not suitable in nutrition of persons sensitized to animal milk protein and/or lactose intolerant. Due to beneficial influence on the human organism of Lactic Acid Bacteria (LAB), there is a constant growth of interest in new products obtained on the way of lactic fermentation, and especially as regards milk-less products that feature pro-healthy and even therapeutic values, provided that their sensory properties are positively perceived by the consumers.

Fermented bevarages based on fruit juices are disclosed in EP 150 8282 A1.

From the description of patent EP 0166238, methods of production of fermented beverages based on fruit juices are known, e.g. apples' (pH 2.9-3.3), grapefruits' (pH 3.0), strawberries' (pH 3.3-3.4) and grapes' (pH 3.4-4.5) juices, that enable overcoming the barriers of the fermentation process, such as low value of active acidity of the substrate or high level of content of phenol and bacteriostatic substances. Fermented fruit drinks are obtained after adapting fruit juices to fermentation, that consists in removing their natural polyphenols during the processes of absorption by way of introducing specific edible or inedible absorbents that are subsequently removed by filtration. In case of some inedible absorbents, after their elimination, a surplus of milk powder is added to increase pH to the value at least 4.5. The prepared preparation is pasteurized at temperature 90°C for 1 min, and then inoculated with a monoculture *Lactobacillus casei* IFO 12004 or *Lactobacillus acidophilus* IFO 3205 cultivated in fruit juice previously treated with milk (removed then by filtration) or in fermented milk; at high population numbers [3.4x10⁸-1.5x10⁹ cfu/mL] of microorganisms in inocula. Beverages obtained in different conventions, with addition of milk or multiple addition of milk at different steps of preparing the fruit matrix for fermentation, depending on the final products storage temperature, maintain after 2 weeks the population numbers of 1.1x10⁷-9.4x10⁸ cfu/mL of microorganisms. In beverages obtained basing on preparations with slight share of milk, used in different functions (absorbent of juice polyphenols; additive supporting fermentation; starter carrier in inoculum) there is a high rate of reduction of microorganism cells number, so that after 1 week of storage of fermented beverage at temperature 8°C, number of population cells in the biotum is 2.0x10³ cfu/mL.

Even though the developed methods of the beverage obtaining influence the possibility to diversify the assortment of fermented fruit beverages, the most frequently recommend method of producing the fruit matrix needed in order to obtain such beverages, consists in preferred application of milk (defatted or casein or sodium caseinate) - basically in order to increase the buffer capacity of the preparation with aforementioned simultaneous elimination of juice polyphenols - what in turn reduces nutritive values of the final products, disturbing their fruity character and eliminating from the group of milk-less products.

Patent EP 0308064 discloses fermented beverages based on tomato products with participation of milk and the methods of their obtaining. According to the invention, tomato juices, purée, pastes or their mixes with ca. 12% suspensions of defatted milk powder, are brought to pH value 4.4-5.3, optimally 4.7-5.1 and sterilized. The obtained preparations are inoculated with the cultures belonging to the *Lactobacillus bulgaricus* and/or *Lactobacillus helveticus* and/or *Streptococcus thermophilus* species, that guarantee obtaining harmonized flavour of fermented drinks with participation of tomato preparations.

Within the last decade, progress of knowledge took place concerning the benefits of lactic fermentation in many additional aspects except for always valued preservation advantages of the process and its valuable sensory effects.

The major product of lactic fermentation and important constituent of fermented fruit beverages is lactic acid. In nutrition, especially of children, the most important thing is the form of lactic acid given in the diet. Since 1974 FAO/WHO [Food and Agricultural Organization/World Health Organization] recommends the adults to limit the consumption of D(-) optical isomer of lactic acid, one of two naturally occurring stereo isomers of lactic acid, to 100 mg per day. In case of fermented products for children nutrition, the only acceptable form of lactic acid is L(+) lactate. Presence of D(-) lactate in fermented products for newborns and children is perceived as highly unwanted to the highest extent. This can be explained by poor potential of utilization of D(-) isomer of lactic acid by child's organism and the risk of occurring of D(-) acidose, particularly in population of children with short bowel syndrome, described in the paper "Probiotic therapy in children with short bowel syndrome and bacterial overgrowth" [Young R., Vanderhoof J.A., Probiotic therapy in children with short bowel syndrome and bacterial overgrowth 1997. Poster at the Annual Meeting of American Gastroenterology Association. May 11-14, Washington DC, USA].

Most of the micro-organisms species applied in the industry for fermentation of food, including many probiotic cultures of *Lactobacillus* genus, produce both, D(-) and L(+) isomers of lactic acid, what is reflected in the composition of the final product of fermentation. They are known from the publication "Lactic Acid Bacteria: Classification and Physiology" [Axelsson L., Lactic Acid Bacteria: Classification and Physiology. In: Lactic Acid Bacteria. Microbial and Functional Aspects (Lactic Acid Bacteria. Functional and Microbiological Aspects). Eds.: Salminen S., Wright von A., Ouvehand A., within "A series of Food Science and Technology". Marcel Dekker, Inc., New York, Basel 2004, pp. 1-66] distinct, dependent on the lactate dehydrogenase (D-nLDH or L-nLDH) availability in the microflora of lactic fermentation, species' predisposition of microorganisms used in the production of fermented food for development of two naturally occurring stereo isomers in variants: exclusively L(+) isomer of lactic acid, exclusively D(-) isomer of lactic acid, approximately equal quantities of both isomers or their mix with predominating content of one of them, but at measurable content of the other. In the cases of fermentations with participation of some of the LAB species, a dependence of induction of lactate racemase formation on the presence of the L(+) lactic acid is known, as a result of which there is a progressing conversion of generated L(+) to D(-) form of lactic acid, and in effect the occurrence of mix of D(-) and L(+) isomers of lactic acid in the fermentation products with growing, in time, share of D(-) isomer in the total content of lactic acid [Sakai K., Fujii N., Chukeatirote E., Racemization of L-lactic acid in pH-swing open fermentation of kitchen refuse by selective proliferation of Lactobacillus plantarum. Journal of Bioscience and Bioengineering 2006, 102, 3, 227-232].

However, the achieved, as a result of fermentation, final output of both isomeric forms of lactic acid, their optical purity and occurring changes in the composition proportions may be influenced also by the following factors: medium composition [Iino T. et al.; The effect of sodium acetate on the activity of L- and D-lactate dehydrogenases in Lactobacillus sakei NRIC 1071T and other lactic acid bacteria. J. Gen. Appl. Microbiology 2003 49(1): 51-58 as well as: Hofvendahl K.&Hahn-Hagerdal B.; L-Lactic acid production from whole wheat flour hydrolysate using strains of Lactobacilli and Lactococci. Enzyme and Microbial Technology 1997, 20, 4, 301-307], physical conditions of incubation [Qing-Jin et al.; Effect of lactic acid bacteria on D(-) and L(+) lactic acid contents of kimchi. Food Science and Biotechnology 2006, 15, 6, 948-953] as well as the method of handling the fermentation products e.g. introduction of additives to soured products [Lutomski L., Importance of lactic acid and its derivatives in diet and therapy. In: Application of lactic acid and its derivatives. Edited by: M. Jankiewicz, PTTZ (Polskie Towarzystwo Technologow Zywności - Polish Association of Food Technologists) - Great Poland Branch, 1995, 59-71].

Except for some of homofermentative strains of relatively anaerobic bacteria belonging to the *Lactobacillus* genus, also absolute anaerobic, homofermentative bifidobacteria are lactic acid producers, that represent physiological microorganisms of intestinal biota. Regardless of their specific direction of metabolism, cultures of the *Lactobacillus plantarum* and *Lactobacillus paracasei* alike bifidobacteria species, can manifest the ability to colonize the intestines thus the advantage of probiotic, therapeutic or prophylactic effects. Probiotics in the gastrointestinal tract act competitively with pathogenic bacteria. Prebiotic substance present in plant raw materials are classed among stimulators of probiotic microorganisms. Combinations of probiotics with prebiotics in foodstuffs, manifesting synergistic action in organism, are called synbiotics [Socha J., Stolarczyk A., Socha P., Role of bifidobacteria in prophylaxis and treatment of selected children diseases. Contemporary Paediatrics, Gastroenterology, Hepatology and Children Nutrition, 2002, 4, 1, 43-47].

The purpose of the invention is development of the production method of fermented fruit beverages without milk constituents - that represent the source of beneficial LABs or LABs and bifidobacteria, with the possibly exclusive share of L(+) isomer in the total content of lactic acid - that enable satisfactory sensory and nutritionally safe application in place of fermented milk in human nutrition, including children; especially of the subjects with allergies to milk protein or lactose intolerant.

The object of the invention is the method of production of a fermented, pro-healthy fruit beverage comprising the steps of:
preparing a fruit preparation for fermentation,
thermal preservation of said fruit preparation,
inoculation of said fruit preparation with a lactic acid bacterial culture; and
fermentation of said inoculated fruit preparation to a pH of no higher value than 4.6;
   said method characterized in that the fruit preparation (i) has a fruit purée content of 25% to 98% by weight, containing of 8% to 12% by weight of extract, (ii) has a non-lactose disaccharide content of 1% to 6% by weight, (iii) has a sodium citrate dihydrate content of 0% to 0.3% by weight, (iv) has a pH of at least 5.0, and (v) is inoculated with a lyophilized culture of a lactic acid bacterium (LAB) selected from a group consisting of *Lactobacillus plantarum* KKP2025p, *Lactobacillus plantarum* KKP2026p and *Lactobacillus paracasei* ssp. *paracasei* KKP2027p, in quantity of 0.001 % to 0.01 % by weight; and
   wherein at a temperature 37°C, said cultures multiplicate in the preparation environment to the level 8.5-9.0 log cfu•g⁻¹, within 8-15 hours.

Preferably, according to the invention a biotum supplement is added to the fermented product in a form of a concentrated lyophilized culture of *Bifidobacteriurn breve* KKP2028p or *Bifidobacterium infantis* KKP2029p strain, in a quantity of 0.01% to 0.1% by weight. Preferably, the method of invention is characterized in that the fruit preparation comprises a banana purée or a melon purée.

According to the invention, the fermentation is carried out at a temperature of 30° C to 43° C. More preferably, the fermentation is carried out at a temperature of 37°C.

Another object of the invention is the strain of *Lactobacillus plantarum* deposited in the Collection of Industrial Microorganisms (IAFB) under the accession number KKP 2025p. Another object of the invention is use of a microbial strain selected from a group consisting of:
a *Lactobacillus plantarum* strain deposited in the Collection of Industrial Microorganisms (IAFB) under the accession number KKP 2025p;
a *Lactobacillus plantarum* strain deposited in the Collection of Industrial Microorganisms (IAFB) under the accession number KKP 2026p;
a *Lactobacillus paracasei* ssp. *paracasei* strain deposited in the Collection of Industrial Microorganisms (IAFB) under the accession number KKP 2027p;
for production of fermented, pro-healthy fruit beverages.

The *Lactobacillus plantarum* KKP 384 strain used in the invention is also deposited in the International Collection of Industrial Microorganisms of the Institute of Agricultural and Food Biotechnology (IAFB). The strains: *Lactobacillus plantarum* JCM 8348 and *Lactobacillus paracasei* ssp. *paracasei* JCM 2769 are taken from the Japan Collection of Microorganisms, *Bifidobacterium breve* ATTC 15700 strain and *Bifidobacterium infantis* ATTC 15697 *Bifidobacterium infantis* ATTC 15697 strain from the American Type Culture Collection.

Said strains are presented in Table I.

**Table I**

| No. | Deposit no. assigned by IAFB | Strain |
|---|---|---|
| 1 | KKP 2025p | *Lactobacillus plantarum* KKP 384 |
| 2 | KKP 2026p | *Lactobacillus plantarum* JCM 8348 |
| 3 | KKP 2027p | *Lactobacillus paracasei* ssp. *paracasei* JCM 2769 |
| 4 | KKP 2028p | *Bifidobacterium breve* ATTC 15700 |
| 5 | KKP 2029p | *Bifidobacterium infantis* ATTC 15697 |

It was found out, that advantageously selected LAB cultures belonging to the *Lactobacillus* genus are able to grow without addition of milk in fruit preparations of relatively high active acidity (pH ~ 5.0), after introduction to them appropriate quantity of cells of selected starter strains. At a temperature 37°C, cultures in question multiplicated their cell populations in the preparation environment to the level 8.5-9.0 log cfu·g⁻¹, within 8-15 h.

Unexpectedly, it was found advantageous, that fruit preparations (banana and melon) prepared according to the method of the invention, enable the starter strain *Lactobacillus paracasei* ssp. *paracasei* JCM2769 to produce almost exclusively the , L(+) isomer of lactic acid.

It was found advantageous, that environment of the fermented fruit beverages (banana and melon), soured with the use of monoculture of *Lactobacillus paracasei* ssp. *paracasei,* after introduction of the bacterial supplement in the form of *Bifidobacterium breve* ATCC 15700 strain or *Bifidobacterium infantes* ATCC 15697 strain into the beverage biotum, does not influence the transformation of the L(+) to D(-) stereo isomer of lactic acid.

Moreover, it was found unexpected and advantageous, that two cultures applied as two-strain set in the invention, *Lactobacillus paracasei* ssp. *paracasei* JCM2769 starter and microbiological supplement of biotum of soured fruit (banana, melon) product, they are capable of cohabitation and maintenance of lactic acid in the form of L(+) stereo isomer exclusively during 28-days' storage of the final product at a temperature 4°C.

In the method according to the invention, fruit preparations were developed of composition providing buffer capacity that enable their souring with LABs without the need of elimination of valuable fruit components, such as polyphenol substances, nor performing vast chemical neutralizing treatments, thus guaranteeing the nutritive and economical benefits. Texture of the preparations provide fluidity of the fermentation products.

Souring of the preparations is achieved at economically justified concentration of starter cultures in the inocula that provide rational duration of the fermentation process, achievement and subsistence in the final products, within 28 days of storage at a temperature 4°C of high population numbers of bacteria that can participate in intestinal biota of consumer.

Fruity, fermented milk-less beverages are directed for consumption especially by the group of people deprived of the option to consume milk products due to intolerance of milk constituents. These fermented fruit beverages, that contain exclusively L(+) isomer of lactic acid, can be used in children's nutrition - especially children sensitized to milk constituents and susceptible of D(-) acidose.

Fermented fruit beverages may, after souring, be complemented with other products, e.g. purées, juices or additional substances, striving to achieve the required sensory profile. The products can be diluted with water, sparkling water or lactic fermentation products of other food matrices. Proper products and additional substances (taste-odour, or texture forming), not limiting the development of preselected starters monocultures, may also be added to the fruit preparations for fermentation.

The invention is closer illustrated by embodiments not limiting its scope.

### Example I

The method of producing of pro-healthy melon beverage containing L(+) isomer of lactic acid exclusively.

Fruit preparation in the form of melon (*Cucumis melo* L.) purée with addition of 2% by weight of saccharose, containing 12.5% by weight of extract was the food matrix to obtain the fermented beverage. The preparation was sterilized at temperature 121°C for 10 min, cooled down to 37°C, and 0.003% by weight of concentrated lyophilized culture *Lactobacillus paracasei* ssp. *paracasei* JCM2769 was added.

The preparation mixed with the starter culture was fermented at temperature 37°C for about 10 hours until souring to pH not higher than 4.6.

*Bifidobacterium infantis* ATCC 15697 culture was added to the fermentation product as a supplement of the starter culture belonging to the *Lactobacillus* genus, in concentrated lyophilized form, in quantity 0.10% by weight.

The fermented melon beverage was poured into glass unitary packages and placed in cold store at temperature 4°C.

During storage under cold conditions, the prohealthy melon beverage was characterized by the change of parameters related to the acidity: drop pH value, increase of titratable acidity and the total content of lactic acid, in the form of L(+) isomer exclusively. During the storage, the beverage manifested flavour specific for milk-less products of lactic fermentation with dominating notes of sour odour and taste. The general sensory profile of the product, characterized by a harmony of sweet and sour notes of taste, relatively fluid, uniform texture, was well evaluated until the end of 28-days' storage within the scope of attributes of flavour (odour and taste) and texture.

Population numbers of bacteria belonging to the *Lactobacillus* or pertaining to the *Bifidobacterium* genera amounted in the fresh product about 8.8 log cfu·g⁻¹ or 8.0 log cfu·g⁻¹ and did not drop below 8.0 or 6.0 log jtk·g⁻¹ after 28 days of cold storage, respectively.

Content of lactic acid amounting about 0.317 g/100g in the fresh product, after 28 days of storage reached the value of 0.725 g/100g, however, the share of L(+) stereo isomer of lactic acid in its total content subsisted at the level no lower than 97% within the full storage period.

### Example II

The method of producing of fermented banana and melon beverages containing the mix of L(+) and D(-) isomers of lactic acid.

Preparation of banana (*Musa paradisiaca* L.) purée containing 10.5% by weight of extract, in the form of 24% by weight of the purée suspension in aqueous solution 4% by weight of saccharose and 0.2% by weight of sodium citrate dihydrate, as well as preparation of melon (*Cucumis melo* L.) purée containing 12.5% by weight of extract after introduction of 2% by weight of saccharose, were the food matrices for obtaining fermented fruit beverages.

Each of the preparations was sterilized at temperature 121°C for 10 min, cooled down to 34°C, 0.001% of concentrated lyophilized culture *Lactobacillus plantarum* (KKP384 - banana preparation; JCM 8348 - melon preparation) was added.

After inoculation, the preparations were fermented at temperature 34° until souring to pH no higher than 4.6; the banana preparation for 13 hours and melon preparation for 10 hours.

The fermented fruit beverages were poured into glass unitary packages and placed in cold store at temperature 4°C.

During storage under cold conditions, both prohealthy beverages were characterized by the change of parameters related to the acidity: drop pH value, increase of titratable acidity and the total content of lactic acid, including L(+) and D(-) isomers of lactic acid.

During cold storage, each of the beverages was characterized by flavour specific to milk-less products of lactic fermentation with dominating notes of sour odour and taste as well as notes typical for the used fruit. The general sensory profile of the products, with a harmony of sweet and sour notes of taste, relatively fluid, uniform texture, was well evaluated until the end of 28-days' storage within the scope of attributes of flavour (odour and taste) and texture.

Population numbers of lactic acid bacteria belonging to the *Lactobacillus* genus amounted in the freshly soured banana beverage 8.8 log cfu·g⁻¹ and in melon beverage 9.1 log cfu·g⁻¹. After 28 days of storage, these values did not drop below 8.1 log cfu·g⁻¹ in banana beverage and 8.8 log·cfu·g⁻¹ in melon beverage.

Content of lactic acid in the obtained banana beverage was 0.220 g/100g and in melon beverage 0.400 g/100g. After 28 days of storage of beverages, the total content of lactic acid reached 0.450 g/100g in banana beverage and 0.830 g/100g in melon beverage. Share of L(+) stereo isomer in the total content of lactic acid in banana and melon drink amounted 43% and 36%, respectively. These values, during the 28-days' cold storage increased to 48% and 43% adequately.

### Example III

The method of producing of pro-healthy banana-melon beverage containing L(+) isomer of lactic acid exclusively.

Preparations in embodiment II: banana and melon, were the food matrices for obtaining fermented fruit beverage.

Each of the preparations was sterilized at temperature 121°C for 10 min, cooled down to 37°C, and 0.005% by weight of concentrated lyophilized culture *Lactobacillus paracasei* ssp. *paracasei* JCM 2769 was added.

After inoculation, the preparations were fermented at temperature 37°C for: 16 hours (banana) and 10 hours (melon), until souring to pH no higher than 4.6.

Both fermented products were mixed aseptically in ratio 1:1.

*Bifidobacterium breve* ATCC 15700 culture was added as a supplement of the starter culture, in concentrated lyophilized form, in quantity 0.10% by weight.

The drink was poured into glass unitary packages and placed in cold store at temperature 4°C.

During storage under cold conditions, prohealthy banana-melon beverage was characterized by the change of parameters related to the acidity: drop pH value, increase of titratable acidity and the total content of lactic acid in the form of L(+) isomer, exclusively.

During cold storage, the beverage was characterized by flavour specific to milk-less products of lactic fermentation with notes of sour odour and taste as well as dominating notes of fruit flavour with advantage of accepted banana note. The general sensory profile of the beverage, with a harmony of sweet and sour notes of taste, banana notes of flavour, relatively fluid, uniform texture, was well evaluated until the end of 28-days' storage within the scope of attributes of flavour (odour and taste) and texture. Population numbers of bacteria belonging to the genera *Lactobacillus* or *Bifidobacterium* amounted in the fresh product about 8.5 log cfu·g⁻¹ or 8.0 log cfu·g⁻¹ and did not drop below 7.8 or 6.0 log jtk·g⁻¹ after 28 days of cold storage, respectively. Content of lactic acid amounting about 0.320 g/100g in the fresh product, after 28 days of storage reached the value of about 0.650 g/100g, however the share of L(+) stereo isomer of lactic acid in its total content in the product sustained at the level close to 97% by weight during complete period of storage.

## Claims

1. A method of production of a fermented, pro-healthy fruit beverage comprising the steps of:
preparing a fruit preparation for fermentation,
thermal preservation of said fruit preparation,
inoculation of said fruit preparation with a lactic acid bacterial culture; and
fermentation of said inoculated fruit preparation to a pH of no higher value than 4.6;
said method **characterized in that** the fruit preparation (i) has a fruit purée content of 25% to 98% by weight, containing 8% to 12% by weight of extract, (ii) has a non-lactose disaccharide content of 1% to 6% by weight, (iii) has a sodium citrate dihydrate content of 0% to 0.3% by weight, (iv) has a pH of at least 5.0, and (v) is inoculated with a lyophilized culture of a lactic acid bacterium (LAB) selected from a group consisting of *Lactobacillus plantarum* KKP2025p, *Lactobacillus plantarum* KKP2026p and *Lactobacillus paracasei* ssp. *paracasei* KKP2027p, in quantity of 0.001 % to 0.01% by weight; and
wherein at a temperature 37°C, said cultures multiplicate in the preparation environment to the level 8.5-9.0 log cfu•g⁻¹, within 8-15 hours.

2. The method according to claim 1, **characterized in that** a biotum supplement is added to the fermented product in a form of a concentrated lyophilized culture of *Bifidobacterium breve* KKP2028p or *Bifidobacterium infantis* KKP2029p strain, in a quantity of 0.01% to 0.1% by weight.

3. The method according to claim 1, **characterized in that** the fruit preparation comprises a banana purée or a melon purée.

4. The method according to claim 1, **characterized in that** the fermentation is carried out at a temperature of 30° C to 43° C.

5. The method according to claim 1, **characterized in that** the fermentation is carried out at a temperature of 37°C.

6. A strain of *Lactobacillus plantarum* deposited in the Collection of Industrial Microorganisms IAFB under the accession number KKP 2025p.

7. Use of a microbial strain selected from a group consisting of:
a *Lactobacillus plantarum* strain deposited in the Collection of Industrial Microorganisms IAFB under the accession number KKP 2025p;
a *Lactobacillus plantarum* strain deposited in the Collection of Industrial Microorganisms IAFB under the accession number KKP 2026p;
a *Lactobacillus paracasei* ssp. *paracasei* strain deposited in the Collection of Industrial Microorganisms IAFB under the accession number KKP 2027p
for production of fermented, pro-healthy fruit beverages.

## Patentansprüche

1. Verfahren zur Herstellung eines fermentierten pro-Gesundheit-Fruchtgetränks, umfassend die Schritte von:
Präparieren einer Fruchtpräparation zur Fermentation,
thermische Konservierung der Fruchtpräparation,
Beimpfung der Fruchtpräparation mit einer Milchsäurebakterienkultur; und
Fermentation der beimpften Fruchtpräparation auf einen pH mit einem nicht höheren Wert als 4,6;
wobei das Verfahren **dadurch gekennzeichnet ist, dass** die Fruchtpräparation (i) einen Gehalt von Fruchtpüree von 25 Gew% bis 98 Gew% aufweist, enthaltend 8 Gew% bis 12 Gew% an Extrakt, (ii) einen nicht-Laktose Disaccharidgehalt von 1 Gew% bis 6 Gew% aufweist, (iii) einen Natriumzitratdihydratgehalt von 0 Gew% bis 0,3 Gew% aufweist, (iv) einen pH von mindestens 5,0 aufweist, und (v) mit einer lyophilisierten Kultur eines Milchsäurebakteriums (LAB) ausgewählt aus einer Gruppe bestehend aus *Luctobacillus plantarum* KKP 2025p, *Luctobacillus plantarum* KKP 2026p und *Luctobacillus paracasei* ssp. *paracasei* KKP 2027p, in einer Menge von 0,001 Gew% bis 0,01 Gew% beimpft wird; und
wobei bei einer Temperatur von 37°C sich die Kulturen in der Präparationsumgebung innerhalb von 8-15 Stunden auf das Niveau von 8,5-9,0 log cfu * g⁻¹ vermehren.

2. Verfahren nach Anspruch 1, **dadurch gekennzeichnet, dass** zu dem fermentierten Produkt ein Biotum-Ergänzungsstoff in einer Form einer konzentrierten lyophilisierten Kultur von Stamm *Bifidobacterium breve* KKP2028p oder *Bifidobacterium infantis* W2029p, in einer Menge von 0,01 Gew% bis 0,1 Gew% hinzugefügt wird.

3. Verfahren nach Anspruch 1, **dadurch gekennzeichnet, dass** die Fruchtpräparation ein Bananenpüree oder ein Melonenpüree umfasst.

4. Verfahren nach Anspruch 1, **dadurch gekennzeichnet, dass** die Fermentation bei einer Temperatur von 30°C bis 43°C durchgeführt wird.

5. Verfahren nach Anspruch 1, **dadurch gekennzeichnet, dass** die Fermentation bei einer Temperatur von 37°C durchgeführt wird.

6. Stamm von *Luctobacillus plantarum* wie hinterlegt in der Sammlung von Industriellen Mikroorganismen IAFB unter der Zugangsnummer KKP 2025p.

7. Verwendung eines Mikrobenstmmes ausgewählt aus einer Gruppe bestehend aus:
einem *Luctobacillus plantarum*-Stamm wie hinterlegt in der Sammlung von Industriellen Mikroorganismen IAFB unter der Zugangsnummer KKP 2025p;
einem *Luctobacillus plantarum*-Stamm wie hinterlegt in der Sammlung von Industriellen Mikroorganismen IAFB unter der Zugangsnummer KKP 2026p;
einem *Luctobacillus paracasei* ssp. *paracasei*-Stamm wie hinterlegt in der Sammlung von Industriellen Mikroorganismen IAFB unter der Zugangsnummer KKP 2027p
zur Herstellung von fermentierten pro-Gesundheits-Fruchtgetränken.

## Revendications

1. Procédé de production d'une boisson aux fruits fermentée bonne pour la santé comprenant les étapes consistant à :
préparer une préparation auxfruits pour une fermentation,
préserver thermiquement ladite préparation aux fruits,
inoculer ladite préparation auxfruits avec une culture de bactérie lactique ; et
fermenter ladite préparation aux fruits inoculée jusqu'à un pH dont la valeur n'excède pas 4,6 ;
ledit procédé est **caractérisé en ce que** la préparation aux fruits (i) a une teneur en purée de fruits de 25 % à 98 % en poids, contenant de 8 % à 12 % en poids d'extrait, (ii) a une teneur en disaccharide non-lactose de 1 % à 6 % en poids, (iii) a une teneur en citrate de sodium dihydraté de 0 % à 0,3 % en poids, (iv) a un pH d'au moins 5,0, et (v) est inoculée avec une culture lyophilisée d'une bactérie lactique (LAB) sélectionnée dans un groupe constitué par *Lactobacillus plantarum* KKP2025p, *Lactobacillus plantarum* KKP2026p et *Lactobacillus paracasei* ssp. *paracasei* KKP2027p, en une quantité de 0,001 % à 0,01 % en poids ; et
dans lequel à une température de 37°C, lesdites cultures se multiplient dans l'environnement de la préparation jusqu'au niveau de 8,5-9,0 log cfu·g⁻¹, en l'espace de 8-15 heures.

2. Procédé selon la revendication 1, **caractérisé en ce qu'**un supplément de biotum est ajouté au produit fermenté sous forme de culture lyophilisée concentrée d'une souche de *Bifidobacterium breve* KKP2028p ou de *Bifidobacterium infantis* KKP2029p, en une quantité de 0,01 % à 0,1 % en poids.

3. Procédé selon la revendication 1, **caractérisé en ce que** la préparation aux fruits comprend une purée de banane ou une purée de melon.

4. Procédé selon la revendication 1, **caractérisé en ce que** la fermentation est réalisée à une température de 30°C à 43°C.

5. Procédé selon la revendication 1, **caractérisé en ce que** la fermentation est réalisée à une température de 37°C.

6. Souche de *Lactobacillus plantarum* déposée dans la collection de micro-organismes industriels IAFB sous le numéro d'accession KKP 2025p.

7. Utilisation d'une souche microbienne sélectionnée dans un groupe constitué par :
une souche de *Lactobacillus plantarum* déposée dans la collection de micro-organismes industriels IAFB sous le numéro d'accession KKP 2025p ;
une souche de *Lactobacillus plantarum* déposée dans la collection de micro-organismes industriels IAFB sous le numéro d'accession KKP 2026p ;
une souche de *Lactobacillus paracasei ssp. paracasei* déposée dans la collection de micro-organismes industriels IAFB sous le numéro d'accession KKP 2027p
pour la production de boissons aux fruits fermentées bonnes pour la santé.
